(19) Europäisches Patentamt
European Patent Office
Office européen des brevets

(11) Veröffentlichungsnummer: **0 281 907**
**A1**

(12) # EUROPÄISCHE PATENTANMELDUNG

(21) Anmeldenummer: **88103118.1**

(22) Anmeldetag: **02.03.88**

(51) Int. Cl.⁴: **C07D 277/34**, C07D 277/36, A01N 43/78

(30) Priorität: **13.03.87 DE 3708201**

(43) Veröffentlichungstag der Anmeldung: **14.09.88 Patentblatt 88/37**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Sasse, Klaus, Dr.**
**Puetzweg 13**
**D-5060 Bergisch Gladbach 2(DE)**
Erfinder: **Fischer, Reiner, Dr.**
**Rembrandtstrasse 15**
**D-4019 Monheim(DE)**
Erfinder: **Hagemann, Hermann, Dr.**
**Kandinskystrasse 52**
**D-5090 Leverkusen 1(DE)**
Erfinder: **Krebs, Andreas, Dr.**
**Am Gartenfeld 70**
**D-5068 Odenthal-Holz(DE)**
Erfinder: **Schwamborn, Michael, Dr.**
**von-Lohe-Strasse 9**
**D-5000 Koeln 80(DE)**
Erfinder: **Santel, Hans-Joachim, Dr.**
**Gruenstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Robert Harry, Dr.**
**Unterdorfstrasse 6a**
**D-4000 Düsseldorf 31(DE)**

(54) **Neue Thiazolylether- und -thioetherderivate.**

(57) Thiazolylderivate der allgemeinen Formel

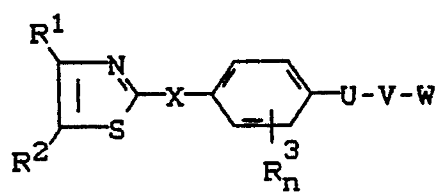

in welcher

EP 0 281 907 A1

$R_1$, $R_2$, $R_3$, X, U, V, W und n die in der Beschreibung angegebene Bedeutung haben, Verfahren zu deren Herstellung und ihre Verwendung als Herbizide.

## Neue Thiazolylether-und -thioetherderivate

Die vorliegende Erfindung betrifft neue substituierte Thiazolyloxy(bzw. thio)aryl-derivate, Verfahren zu ihrer Herstellung und ihre Verwendung als Herbizide.

Es ist bereits bekannt, daß bestimmte Carbonsäureanilide bzw. Benzoesäurederivate herbizide Eigenschaften besitzen (vgl. R. Wegler "Chemie der Pflanzenschutz und Schädlingsbekämpfungsmittel" Bd. 2, Seiten 311-314 bzw. 289-295; Bd. 5 Seiten 209-217, Springer Verlag, Berlin 1970/1977, DE 3 422 007). Ihre Wirkung ist jedoch unter bestimmten Bedingungen, z.B. bei niedrigen Aufwandmengen nicht immer befriedigend. Ebenso läßt die Selektivität in manchen Fällen zu wünschen übrig.

Ferner ist bekannt, daß bestimmte Alkylheterooxyarylderivate herbizide Eigenschaften besitzen (vgl. T. Jojima et al. Agr. Biol. Chem. 30, (9) 866 ff (1966) bzw. JA-OS 9474/1967). Die herbizide Potenz dieser Stoffe ist jedoch unter praxisrelevanten Bedingungen nicht immer ausreichend.

Es wurden nun neue Thiazolylderivate der allgemeinen Formel (I) gefunden,

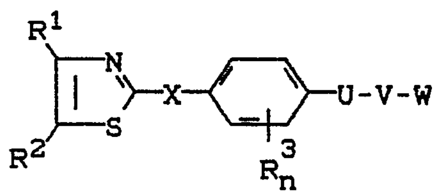

(I)

in welcher

$R^1$ für Wasserstoff oder Alkyl steht,

$R^2$ für Wasserstoff oder Alkyl steht,

mit der Maßgabe, daß $R^1$ und $R^2$ nicht gleichzeitig für Wasserstoff stehen,

X für Sauerstoff oder Schwefel steht,

$R^3$ für Wasserstoff, Halogen, Nitro, Amino, Cyano, Alkyl, Halogenalkyl, Alkoxy oder Halogenalkoxy steht, wobei die Reste $R^3$ gleich oder verschieden sein können,

n für eine Zahl von 1 bis 4 steht

U für Sauerstoff oder Schwefel steht,

V für

eine 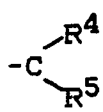 -Gruppe

steht, wobei

$R^4$ und $R^5$ unabhängig voneinander für Wasserstoff, Alkyl, Alkoxyalkyl oder Halogenalkyl steht,

W für einen Rest

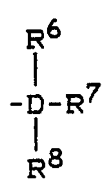 , 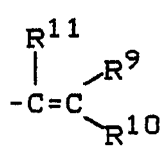 oder 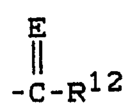

steht, wobei

D für Kohlenstoff oder Silicium steht,

E für Sauerstoff oder Schwefel steht,

$R^6$, $R^{11}$ und $R^{12}$ unabhängig voneinander für Wasserstoff, Halogen, Alkyl oder Halogenalkyl stehen,

$R^7$ für Wasserstoff, Halogen, gegebenenfalls durch Halogen substituiertes Alkyl, Alkoxy, Alkoxyalkyl oder

Alkylthio steht,

$R^8$ für Halogen, einen gegebenenfalls durch Halogen substituierten gesättigten oder ungesättigten aliphatischen Rest mit 1 bis 6 Kohlenstoffatomen, Alkoxy, Alkoxyalkyl, Alkylthio, Formyl, Alkylcarbonyl, Alkoxycarbonyl, offenkettiges oder cyclisches Acetal, offenkettiges oder cyclisches Thioacetal steht, oder

$R^7$ und $R^8$ gemeinsam mit dem Kohlenstoff, an das sie gebunden sind, einen 3 bis 8 gliedrigen Ring bilden, der außer Kohlenstoff auch Sauerstoff und/oder Schwefel und/oder Stickstoff als Ringglieder enthalten kann und der gegebenenfalls durch Halogen, Alkyl, Alkoxy oder Alkoxyalkyl substituiert sein kann und welcher eine oder mehrere Doppelbindungen enthalten kann, oder

$R^6$, $R^7$ und $R^8$ gemeinsam mit dem Kohlenstoffatom, an das sie gebunden sind, für einen Rest der Struktur

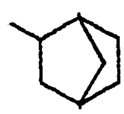

oder

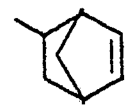

stehen, und

$R^9$ und $R^{10}$ unabhängig voneinander für Wasserstoff, Halogen, Alkyl oder Halogenalkyl stehen.

Enthalten Verbindungen der allgemeinen Formel (I) ein asymmetrisches Kohlenstoffatom, so können sie auch in verschiedenen enantiomeren Formen vorliegen. Die Erfindung betrifft sowohl die möglichen einzelnen Isomeren als auch Gemische der Isomeren.

Ein asymmetrisches Zentrum liegt vor, wenn V für die

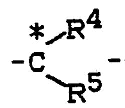 -Gruppe

steht und $R^4$ und $R^5$ unterschiedliche Bedeutung haben, wobei das asymmetrische C-Atom, durch * gekennzeichnet, die R, S oder R/S (Racemat)-Konfiguration aufweisen kann.

Das zweite asymmetrische Zentrum liegt vor, wenn W für

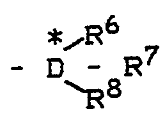

und D für Kohlenstoff steht und $R^6$, $R^7$ und $R^8$ unterschiedliche Bedeutung haben, wobei auch das zweite asymmetrische C-Atom die R, S oder R/S (Racemat)-Konfiguration aufweisen kann.

Weiterhin wurde gefunden, daß man die 2-Thiazolylether und -thioetherderivate der allgemeinen Formel (I) erhält, wenn man

A) Verbindungen der allgemeinen Formel (II),

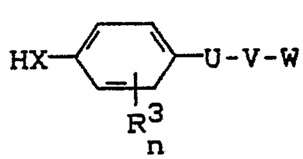

(II)

in welcher

X, $R^3$, U, V, W und n die oben angegebene Bedeutung haben,

mit Verbindungen der allgemeinen Formel (III),

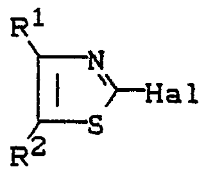

(III)

in welcher

$R^1$ und $R^2$ die oben genannte Bedeutung haben und

Ha1 für Halogen steht,

in Gegenwart eines Verdünnungsmittels und gegebenenfalls in Gegenwart eines Säurebindemittels umsetzt

oder wenn man

B) Verbindungen der allgemeinen Formel (IV),

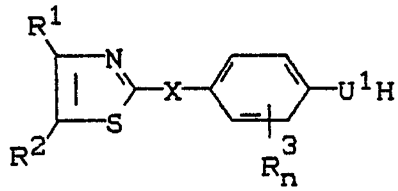

(IV)

in welcher

$R^1$, $R^2$, X, $R^3$ und n die oben angegebene Bedeutung haben

und $U^1$ für Sauerstoff steht,

mit Verbindungen der allgemeinen Formel (V)

L - V - W (V)

in welcher

v und W die oben angegebene Bedeutung haben und

L für Halogen, Alkylsulfonyloxy mit jeweils 1 bis 4 Kohlenstoffatomen, Halogenalkylsulfonyloxy mit jeweils 1 bis 4 Kohlenstoffatomen oder Phenyl-oder substituiertes Phenylsulfonyloxy steht,

in Gegenwart eines Verdünnungsmittels und gegebenenfalls in Gegenwart eines Säurebindemittels umsetzt.

Schließlich wurde gefunden, daß sich die neuen 2-Thiazolylether und -thioether der allgemeinen Formel (I) durch eine sehr gute herbizide Wirksamkeit auszeichnen.

Überraschenderweise besitzen die erfindungsgemäßen 2-Thiazolylether und -thioether der allgemeinen Formel (I) wesentlich bessere herbizide Eigenschaften als konstitutionell ähnliche vorbekannte Stoffe.

Die Kohlenstoffketten sind in den Definitionen jeweils geradlinig oder verzweigt; Halogen steht jeweils für Fluor, Chlor, Brom oder Jod.

Die erfindungsgemäßen Thiazolylether und -thioether sind durch die allgemeine Formel (I) allgemein definiert. Bevorzugt sind Verbindungen der allgemeinen Formel (I), bei welchen

$R^1$ für Wasserstoff oder $C_1$-$C_4$-Alkyl steht,

$R^2$ für Wasserstoff oder $C_1$-$C_4$-Alkyl steht,

mit der Maßgabe, daß $R^1$ und $R^2$ nicht gleichzeitig für Wasserstoff stehen,

X für Sauerstoff oder Schwefel steht,

$R^3$ für Wasserstoff, Halogen, $C_1$-$C_4$-Alkyl, $C_1$-$C_4$-Halogenalkyl, $C_1$-$C_4$-Alkoxy oder Halogen-$C_1$-$C_4$-alkoxy steht, wobei die Reste $R^3$ gleich oder verschieden sein können,

n für eine Zahl von 1 bis 4 steht,

U für Sauerstoff oder Schwefel steht,

V für

eine 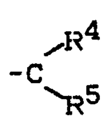 -Gruppe

steht, wobei

$R^4$ und $R^5$ unabhängig voneinander jeweils für Wasserstoff, $C_1$-$C_4$-Alkyl, $C_1$-$C_4$-Alkoxy-$C_1$-$C_4$-alkyl oder Halogen-$C_1$-$C_4$-alkyl stehen,

W für einen Rest

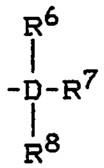 , 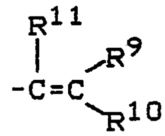 oder 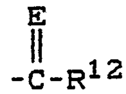

steht, wobei

D für Kohlenstoff oder Silicium steht,

E für Sauerstoff oder Schwefel steht,

$R^6$, $R^{11}$ und $R^{12}$ unabhängig voneinander für Wasserstoff, Halogen, $C_1$-$C_6$-Alkyl oder Halogen-$C_1$-$C_6$-alkyl stehen,

$R^7$ für Wasserstoff, Halogen, $C_1$-$C_6$-Alkyl, $C_1$-$C_6$-Alkoxy, $C_1$-$C_6$-Alkoxy-$C_1$-$C_6$-alkyl, $C_1$-$C_6$-Alkylthio oder Halogen-$C_1$-$C_6$-alkyl steht,

$R^8$ für Halogen, $C_1$-$C_6$-Alkyl, $C_1$-$C_6$-Alkoxy, $C_1$-$C_6$-Alkoxy-$C_1$-$C_6$-alkyl, $C_1$-$C_6$-Alkylthio, Formyl, $C_1$-$C_6$-Alkylcarbonyl, $C_1$-$C_6$-Alkoxycarbonyl, $C_2$-$C_6$-Alkenyl, $C_2$-$C_6$-Alkinyl, Halogen-$C_1$-$C_6$-alkyl, Halogen-$C_2$-$C_6$-alkenyl, offenkettiges Ketal, cyclisches Ketal mit 5 oder 6 Ringgliedern, offenkettiges Acetal, cyclisches Acetal mit 5 oder 6 Ringgliedern, offenkettiges Thioacetal oder cyclisches Thioacetal mit 5 oder 6 Ringgliedern steht oder

$R^7$ und $R^8$ gemeinsam mit dem Kohlenstoffatom, an das sie gebunden sind, einen 5-oder 6-gliedrigen Ring bilden, der außer Kohlenstoff auch 1 oder 2 Sauerstoffatome und/ oder Schwefelatome und/oder Stickstoffatome als Ringglieder enthalten kann und der durch Fluor, Chlor, Brom, Jod, $C_1$-$C_4$-Alkyl, $C_1$-$C_4$-Alkoxy und/oder $C_1$-$C_4$-Alkoxy-$C_1$-$C_4$-alkyl substituiert sein kann und welcher eine oder zwei Doppelbindungen enthalten kann oder

$R^6$, $R^7$ und $R^8$ gemeinsam mit dem Kohlenstoffatom, an das sie gebunden sind, für einen Rest der Struktur

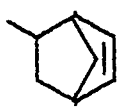 oder 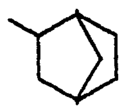

stehen, und

$R^9$ und $R^{10}$ unabhängig voneinander für Wasserstoff, Halogen, $C_1$-$C_6$-Alkyl oder Halogen-$C_1$-$C_6$-alkyl stehen.

Bevorzugt sind auch die optisch aktiven Verbindungen der allgemeinen Formel (I).

Besonders bevorzugt sind Verbindungen der allgemeinen Formel (I), bei welchen

$R^1$ für Wasserstoff, Methyl oder Ethyl steht,

$R^2$ für Wasserstoff, Methyl oder Ethyl steht,

mit der Maßgabe, daß $R^1$ und $R^2$ nicht gleichzeitig für Wasserstoff stehen,

X für Sauerstoff oder Schwefel steht,

$R^3$ für Wasserstoff, Halogen, Methyl, Ethyl, Methoxy, Ethoxy, Halogen-$C_1$-$C_2$-alkyl oder Halogen-$C_1$-$C_2$-alkoxy steht,

wobei die Reste $R^3$ gleich oder verschieden sein können,

n für eine Zahl von 1-4 steht,

U für Sauerstoff oder Schwefel steht,

V für

eine 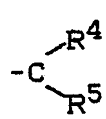 -Gruppe

steht, wobei

$R^4$ und $R^5$ unabhängig voneinander jeweils für Wasserstoff, Methyl, Ethyl, Methoxyethyl, Methoxyethyl,

Ethoxymethyl, Ethoxyethyl oder Halogen-$C_1$-$C_2$-alkyl stehen,
W für einen Rest

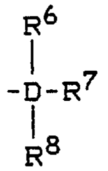 , 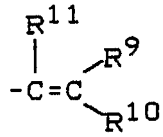 oder 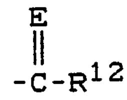

steht, wobei
D für Kohlenstoff oder Silicium steht,
E für Sauerstoff oder Schwefel steht,
$R^6$, $R^{11}$ und $R^{12}$ unabhängig voneinander für Wasserstoff, Fluor, Chlor, Brom, Jod, $C_1$-$C_4$-Alkyl oder Halogen-$C_1$-$C_4$-alkyl steht,
$R^7$ für Wasserstoff, Fluor, Chlor, Brom, Jod, $C_1$-$C_4$-Alkyl, $C_1$-$C_4$-Alkoxy, $C_1$-$C_4$-Alkoxy-$C_1$-$C_4$-alkyl, $C_1$-$C_4$-Alkylthio oder Halogen-$C_1$-$C_4$-alkyl steht,
$R^8$ für Fluor, Chlor, Brom, Jod, $C_1$-$C_4$-Alkyl, $C_1$-$C_4$-Alkoxy, $C_1$-$C_4$-Alkoxy-$C_1$-$C_4$-alkyl, $C_1$-$C_4$-Alkylthio, Formyl, $C_1$-$C_4$-Alkylcarbonyl, $C_1$-$C_4$-Alkoxycarbonyl, $C_2$-$C_4$-Alkenyl, $C_2$-$C_4$-Alkinyl, Halogen-$C_1$-$C_4$-alkyl, Halogen-$C_2$-$C_4$-alkenyl, offenkettiges Acetal, cyclisches Acetal mit 5 oder 6 Ringgliedern, offenkettiges Thioacetal oder cyclisches Thioacetal mit 5 oder 6 Ringgliedern steht oder
$R^7$ und $R^8$ gemeinsam mit dem Kohlenstoffatom, an das sie gebunden sind, einen 5-oder 6-gliedrigen Ring bilden, der außer Kohlenstoff auch 1 oder 2 Sauerstoffatome und/oder Schwefelatome und/oder Stickstoffatome als Ringglieder enthalten kann und der durch Fluor, Chlor, Brom und Jod, Methyl, Ethyl, Methoxy, Ethoxy, Methoxymethyl, Methoxyethyl, Ethoxymethyl oder Ethoxyethyl substituiert sein kann und welcher eine oder zwei Doppelbindungen enthalten kann, oder
$R^6$, $R^7$ und $R^8$ gemeinsam mit dem Kohlenstoffatom, an das sie gebunden sind, für einen Rest der Struktur

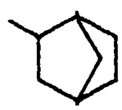 oder 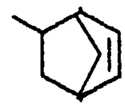

stehen, und
$R^9$ und $R^{10}$ unabhängig voneinander für Wasserstoff, Halogen, $C_1$-$C_2$-Alkyl oder Halogen-$C_1$-$C_2$-alkyl stehen.

Besonders bevorzugt sind auch die optisch aktiven Verbindungen der allgemeinen Formel (I).

Ganz besonders bevorzugt sind diejenigen Verbindungen der allgemeinen Formel (I), in denen
$R^1$ für Wasserstoff, Methyl oder Ethyl steht,
$R^2$ für Wasserstoff, Methyl oder Ethyl steht,
mit der Maßgabe, daß $R^1$ und $R^2$ nicht gleichzeitig für Wasserstoff stehen,
X für Sauerstoff oder Schwefel steht,
$R^3$ für Wasserstoff, Fluor, Chlor, Brom, Methyl, Ethyl, Methoxy, Ethoxy, Trifluormethyl oder Trifluormethoxy steht,
wobei die Reste $R^3$ gleich oder verschieden sein können,
n für eine Zahl von 1-4 steht,
U für Sauerstoff oder Schwefel steht,
V für

eine 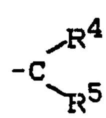 -Gruppe

steht,
$R^4$ und $R^5$ unabhängig voneinander jeweils für Wasserstoff, Methyl, Ethyl, Methoxymethyl, Methoxyethyl, Ethoxymethyl, Ethoxyethyl oder Halogen-$C_1$-$C_2$-alkyl stehen,
W für einen Rest

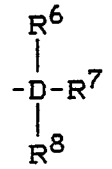 , 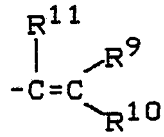 oder 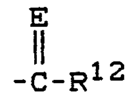

steht,
D für Kohlenstoff oder Silicium steht,
E für Sauerstoff oder Schwefel steht,
$R^6$, $R^{11}$ und $R^{12}$ unabhängig voneinander für Wasserstoff, Fluor, Chlor,Methyl, Ethyl, n-Propyl, iso-Propyl, n-, iso-, sec.-oder tert.-Butyl oder Halogen-$C_1$-$C_2$-alkyl steht,
$R^7$ für Wasserstoff, Fluor, Chlor, Methyl, Ethyl, n-Propyl, iso-Propyl, n-, iso-oder sec.-Butyl, n-,iso-oder sec.-Butoxy, Methoxymethyl, Methoxyethyl, Methoxy-n-propyl, Methoxy-iso-propyl, Ethoxymethyl, Ethoxyethyl, Ethoxy-n-propyl, Ethoxy-iso-propyl,
n-Propoxymethyl, n-Propoxyethyl, n-Propoxy-n-propyl, n-Propoxy-iso-propyl, Methylthio, Ethylthio, Isopropylthio oder Halogen-$C_1$-$C_2$-alkyl steht,
$R^8$ für Fluor, Chlor, $C_1$-$C_4$-Alkyl, $C_1$-$C_4$-Alkoxy, $C_1$-$C_4$-Alkoxy-$C_1$-$C_4$-alkyl, $C_1$-$C_4$-Alkylthio, $C_1$-$C_4$-Alkylcarbonyl, $C_1$-$C_4$-Alkoxycarbonyl, Formyl, $C_2$-$C_4$-Alkenyl, $C_2$-$C_4$-Alkinyl, Halogen-$C_1$-$C_4$-alkyl, Halogen-$C_2$-$C_4$-alkenyl, Halogen-$C_2$-$C_4$-alkinyl, offenkettiges Acetal, cyclisches Acetal mit 5 oder 6 Ringgliedern, offenkettiges Thioacetal oder cyclisches Thioacetal mit 5 oder 6 Ringgliedern für oder offenkettiges Ketal oder cyclisches Ketal mit 5 oder 6 Ringgliedern steht,
$R^7$ und $R^8$ gemeinsam mit dem Kohlenstoffatom, an das sie gebunden sind, einen 5-oder 6-gliedrigen Ring bilden, der außer Kohlenstoff auch Sauerstoff und/oder Schwefel und/oder Stickstoff als Ringglieder enthalten kann und der durch Fluor, Chlor, Methyl, Ethyl, Methoxy, Ethoxy, Methoxymethyl, Ethoxymethyl oder Ethoxyethyl substituiert sein kann und welcher eine oder zwei Doppelbindungen enthalten kann oder
$R^6$, $R^7$ und $R^8$ gemeinsam mit dem Kohlenstoffatom, an das sie gebunden sind, für einen Rest der Struktur

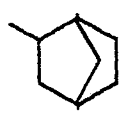 oder 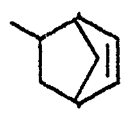 stehen, und

$R^9$ und $R^{10}$ unabhängig voneinander für Wasserstoff, Halogen, $C_1$-$C_2$-Alkyl oder Halogen-$C_1$-$C_2$-alkyl stehen.

Ganz besonders bevorzugt sind auch die optisch aktiven Verbindungen der allgemeinen Formel (I).

Verwendet man beispielsweise 4,5-Dimethyl-2-chlorthiazol und 4-Neopentyloxy-thiophenol als Ausgangsstoffe, so läßt sich der Reaktionsablauf des erfindungsgemäßen Verfahrens (A) durch folgendes Formelschema darstellen:

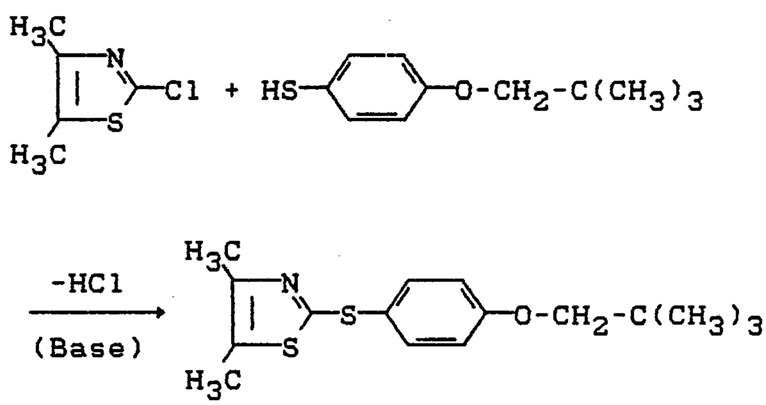


Verwendet man beispielsweise 4-(4,5-Dimethyl-2-thiazolylthio)-phenol und Isobutyltosylat als Ausgangsstoffe, so läßt sich der Reaktionsablauf des erfindungsgemäßen Verfahrens (B) durch das folgende Formelschema darstellen:

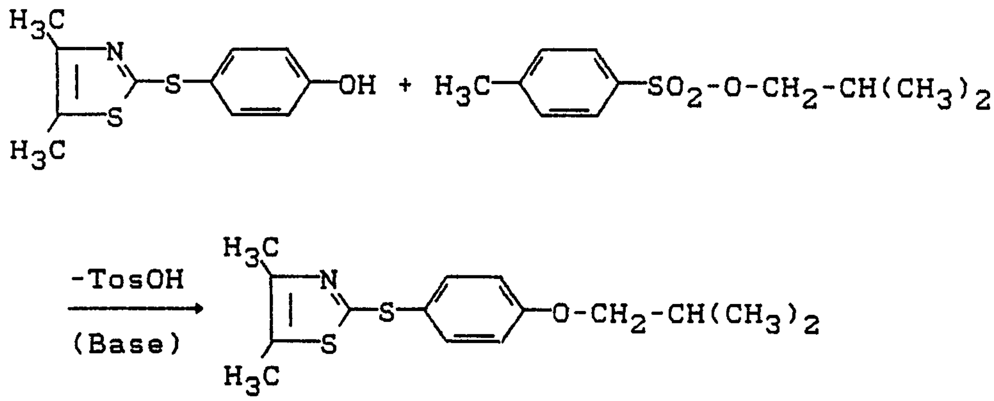


Die zur Durchführung des erfindungsgemäßen Verfahrens (A) als Ausgangsstoffe benötigten (Thio)-phenol-Derivate sind durch die Formel (II) allgemein definiert. In dieser allgemeinen Formel (II) haben X, $R^3$, U, V, W und n vorzugsweise diejenigen Bedeutungen, die bereits bei der Beschreibung der erfindungsgemäßen Stoffe der Formel (I) vorzugsweise für die Substituenten genannt wurden.

Die Thiophenol-Derivate der allgemeinen Formel (II) sind teilweise bekannt oder lassen sich nach bekannten Verfahren in einfacher Weise synthetisieren [vgl. z.B. Coll. Czech. Chem. Commun., 29, 2161 (1964)] und sind zum Teil Inhalt eines eigenen, älteren Schutzbegehrens.

Man erhält die Thiophenol-Derivate der allgemeinen Formel (II), in der X für Schwefel steh wenn man

a) Amino-(thio)phenol-Derivate der allgemeinen Formel (VI),

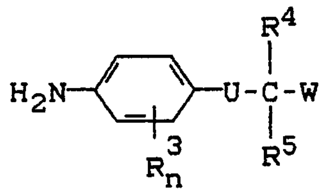
(VI)

in welcher
$R^3$, $R^4$, $R^5$, U, W und n die oben angegebene Bedeutung haben,
in einer 1. Stufe mit einem Nitrierungsmittel, gegebenenfalls in Gegenwart eines Verdünnungsmittels und gegebenenfalls in Gegenwart eines Reaktionshilfsmittels diazotiert, das Daizotierungsprodukt in einer 2. Stufe in Gegenwart eines Verdünnungsmittels und in Gegenwart einer Base mit Alkalixanthogenat umsetzt und anschließend das Reaktionsprodukt in einer 3. Stufe in Gegenwart eines Verdünnungsmittels mit Alkalihydroxid erhitzt,
oder wenn man
b) Benzolsulfochloride der allgemeinen Formel (VII),

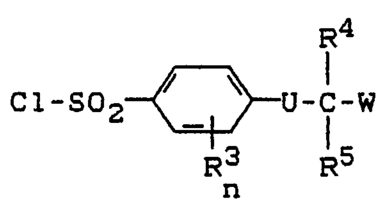
(VII) .

in welcher
$R^3$, $R^4$, $R^5$, U, W und n die oben angegebene Bedeutung haben,
mit Reduktionsmitteln gegebenenfalls in Gegenwart eines Verdünnungsmittels und gegebenenfalls in Gegenwart eines Reaktionshilfsmittels reduziert.

Die Amino-(thio)phenol-Derivate der allgemeinen Formel (VI) erhält man beispielsweise, wenn man Nitro-(thio)-phenol-Derivate der allgemeinen Formel (VIII),

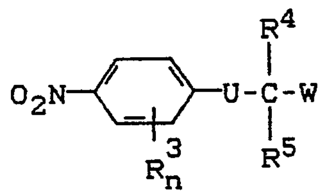

(VIII)

in welcher
$R^3$, $R^4$, $R^5$, U, W und n die oben angegebene Bedeutung haben,
mit Raney-Nickel in Gegenwart eines Verdünnungsmittels bei Drucken von 60 bis 90 bar und bei Temperaturen zwischen 20 und 100°C hydriert.

Die Nitro-(thio)phenol-Derivate der allgemeinen Formel (VIII) sind bekannt und lassen sich nach bekannten Methoden herstellen, indem man beispielsweise 4-Halogennitrobenzole der allgemeinen Formel (IX),

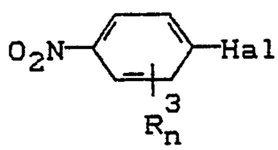

(IX)

in welcher
$R^3$ und n die oben angegebene Bedeutung haben und Hal für Halogen steht,
mit (Thio)Alkoholen der allgemeinen Formel (X),

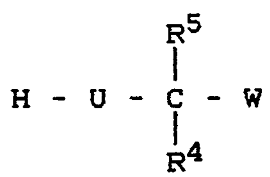

(X)

in welcher
$R^4$, $R^5$, U und W die oben angegebene Bedeutung haben,
in Gegenwart eines Verdünnungsmittels, wie beispielsweise Dimethylsulfoxid, N-Methylpyrrolidon oder Sulfolan und in Gegenwart eines Säurebindemittels, wie beispielsweise Natriumhydrid, Natrium-und Kaliumhydroxid oder Natrium-oder Kalium-alkoholat, bei Temperaturen zwischen +20 und +200°C umsetzt.
Die 4-Halogennitrobenzole der allgemeinen Formel (IX) und die (Thio)Alkohole der allgemeinen Formel (X) sind bekannte Verbindungen oder lassen sich nach bekannten Methoden herstellen.

Die Phenolderivate der allgemeinen Formel (XI),

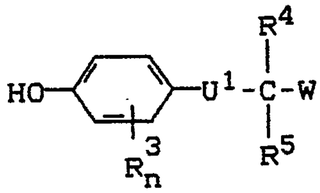

(XI)

in welcher
$R^3$, $R^4$, $R^5$, W und n die oben angegebene Bedeutung haben und $U^1$ für Sauerstoff steht,
sind teilweise bekannt oder lassen sich nach bekannten Verfahren in einfacher Weise darstellen, wenn man Phenolderivate der allgemeinen Formel (XII),

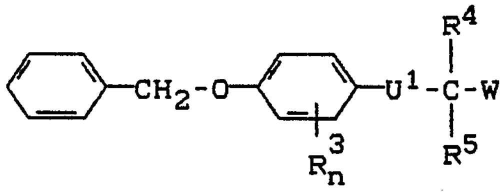

(XII)

in welcher
$R^3$, $R^4$, $R^5$, W und n die oben angegebene Bedeutung haben und $U^1$ für Sauerstoff steht,
mit Pd/C oder $PtO_2$ in Gegenwart eines Verdünnungsmittels bei Drucken von 1 bis 90 bar und bei Temperaturen zwischen 20°C und 100°C hydriert.

Die Phenolderivate der allgemeinen Formel (XII) sind bekannte oder lassen sich nach bekannten Methoden herstellen, indem man beispielsweise Hydrochinonmonobenzylether der allgemeinen Formel (XIII),

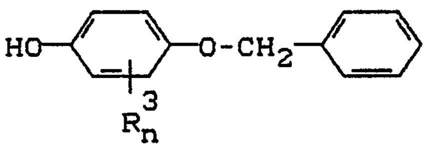

(XIII)

in welcher
$R^3$ und n die oben angegebene Bedeutung haben,
mit Verbindungen der allgemeinen Formel (V),

L - V - W (V)

in welcher
V und W die oben angegebene Bedeutung haben und
L für Halogen, Alkyl-oder Arylsulfonat steht,
in Gegenwart eines Verdünnungsmittels, wie beispielsweise Dimethylsulfoxid, N-Methylpyrrolidon oder Sulfolan und in Gegenwart eines Säurebindemittels, wie beispielsweise Natriumhydrid, Natrium-und Kaliumhydroxid oder Natrium-und Kaliumalkoholat bei Temperaturen zwischen 20°C und 200°C umsetzt.

Die zur Durchführung des erfindungsgemäßen Verfahrens (A) weiterhin als Ausgangsstoffe benötigten Thiazol-Derivate sind durch die Formel (III) allgemein definiert. In dieser allgemeinen Formel (III) stehen die Reste $R^1$ und $R^2$ vorzugsweise für diejenigen Reste, die bereits in der Beschreibung der erfindungsgemäßen Stoffe der allgemeinen Formel (I) als bevorzugt für diese Substituenten genannt wurden. Hal steht vorzugsweise für Fluor, Chlor und Brom.

Beispielhaft seien die folgenden Thiazolyl-Derivate der allgemeinen Formel (III) genannt:
4-Methyl-2-chlor-thiazol
5-Methyl-2-chlor-thiazol
4,5-Dimethyl-2-chlor-thiazol
4-Ethyl-5-methyl-2-chlor-thiazol
5-Methyl-5-ethyl-2-chlor-thiazol
4,5-Diethyl-2-chlor-thiazol.

Die Thiazolderivate der allgemeinen Formel (III) sind bekannt oder lassen sich im Prinzip nach einfachen Methoden darstellen.
Lit. G. Vernin V. J. Metzger, Bull. Soc. Chim. France 2498 (1963).

Die zur Durchführung des erfindungsgemäßen Verfahrens (B) als Ausgangsstoffe benötigten Thiazolyl-(thio)phenole sind durch die allgemeine Formel (IV) definiert. In der allgemeinen Formel (IV) stehen $R^1$, $R^2$, $R^3$, X und n vorzugsweise bzw. besonders bevorzugt für diejenigen Reste, die bereits bei der Beschreibung der erfindungsgemäßen Stoffe der Formel (I) als bevorzugt bzw. besonders bevorzugt für die Substituenten genannt wurden und $U^1$ für Sauerstoff.

Die Verbindungen der allgemeinen Formel (IV),

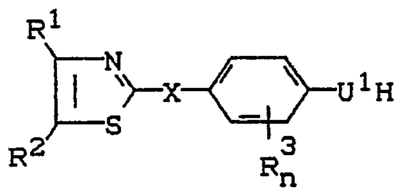

(IV)

in welcher
$R^1$, $R^2$, $R^3$, X und n die oben angegebene Bedeutung haben und $U^1$ für Sauerstoff steht, sind neu und ebenfalls Gegenstand der vorliegenden Erfindung.

Als Beispiele für Thiazolyl-(thio)phenole der allgemeinen Formel (IV ) seien genannt:
4-(4-Methyl-2-thiazolyloxy)-phenol
4-(4-Methyl-2-thiazolylthio)-phenol
4-(5-Methyl-2-thiazolyloxy)-phenol
4-(5-Methyl-2-thiazolylthio)-phenol
4-(4,5-Dimethyl-2-thiazolyloxy)-phenol
4-(4,5-Dimethyl-2-thiazolylthio)-phenol
4-(4-Ethyl-5-methyl-2-thiazolyloxy)-phenol
4-(4-Ethyl-5-methyl-2-thiazolylthio)-phenol
4-(4-Methyl-5-ethyl-2-thiazolyloxy)-phenol
4-(4-Methyl-5-ethyl-2-thiazolylthio)-phenol
4-(4,5-Diethyl-2-thiazolyloxy)-phenol
4-(4,5-Diethyl-2-thiazolylthio)-phenol.

So erhält man beispielsweise Thiazolyl-(thio)phenole der allgemeinen Formel (IV ), wenn man
C) 2-Halogen-thiazol-Derivate der allgemeinen Formel (III)

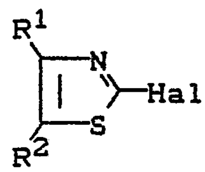

(III)

in welcher
$R^1$ und $R^2$ die obengenannte Bedeutung haben und
Hal für Halogen, insbesondere Fluor, Chlor und Brom steht,
mit Verbindungen der allgemeinen Formel (XIV),

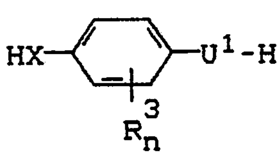

(XIV)

in welcher
$R^3$, $U^1$, X und n die oben angegebene Bedeutung haben,
in Gegenwart eines Lösungsmittels und in Gegenwart eines Säurebindemittels umsetzt.

Die im obigen Verfahren (C) als Ausgangsstoffe benötigten 2-Halogen-thiazole sind durch die Formel (III) allgemein definiert.

In dieser Formel haben $R^1$ und $R^2$ vorzugsweise diejenigen Bedeutungen, die bereits im Zusammenhang mit der Beschreibung der erfindungsgemäßen Stoffe der allgemeinen Formel (I) genannt wurden. Hal steht vorzugsweise für Fluor, Chlor oder Brom. Die Verbindungen der allgemeinen Formel (III) sind bekannt und lassen sich in einfacher Weise darstellen (G. Vernin v. J. Metzger, Bull. Chim. Soc. France 2498 (1963)-).

Die bei dem Verfahren (C) weiterhin benötigten Edukte sind durch die Formel (XIV) allgemein definiert. In dieser Formel haben $R^3$, $U^1$, X und n vorzugsweise diejenigen Bedeutungen, die bereits im Zusammenhang mit der Beschreibung der erfindungsgemäßen Stoffe der allgemeinen Formel (VI) vorzugsweise für die Reste bzw. diese Index genannt wurden.

Die Verbindungen der allgemeinen Formel (XIV) sind bekannt oder lassen sich nach im Prinzip bekannten Methoden in einfacher Weise darstellen.

Als Säurebindemittel können bei der Durchführung des Verfahrens (C) alle üblicherweise für derartige Umsetzungen verwendbaren Säureakzeptoren verwendet werden, vorzugsweise Alkali-und Erdalkalioxide, -hydroxide und -carbonate, ferner Alkali-alkoholate, -amide und -hydride.

Als Verdünnungsmittel können bei der Durchführung des Verfahrens (C) alle üblichen inerten organischen Solven tien verwendet werden. Vorzugsweise Ether, wie Dioxan, Tetrahydrofuran, Glykoldimethylether, sowie Nitrile, beispielsweise Acetonitril und auch stark polare Solventien, wie Dimethylformamid, Dimethylsulfoxid, Sulfolan und N-Methylpyrrolidon.

Die Reaktionstemperaturen können bei der Durchführung des Verfahrens (C) innerhalb eines größeren Temperaturbereiches variiert werden und liegen im allgemeinen zwischen 0°C und 220°C, vorzugsweise 50°C und 180°C.

Die Umsetzung nach dem Verfahren (C) wird im allgemeinen unter Normaldruck vorgenommen.

Bei der Durchführung des Verfahrens (C) setzt man die Ausgangsstoffe der allgemeinen Formeln (III) und (XIV) im allgemeinen in angenähert äquimolaren Mengen um. Es ist jedoch auch möglich, die eine oder andere Komponente in einem größeren Überschuß zu verwenden. Die Aufarbeitung erfolgt nach den üblichen Methoden.

Die bei dem Verfahren (B) weiterhin als Ausgangsstoffe benötigten Verbindungen sind durch die Formel (V) allgemein definiert.

L - V - W (V)

In dieser Formel haben V und W vorzugsweise diejenigen Bedeutungen, die bereits bei der Beschreibung der erfindungsgemäßen Stoffe der allgemeinen Formel (I) als be vorzugt für die Substituenten genannt wurden. L steht vorzugsweise für Chlor, Brom, Jod, Methylsulfonyl, Ethylsulfonyl, Phenylsulfonat, Tosylat oder Mesylat.

Beispielhaft seien die folgenden Verbindungen der allgemeinen Formel (V) genannt:

Isobutyl-tosylat
Neopentyl-tosylat
2.2-Dimethyl-1-butyl-tosylat
2.2-Dimethyl-1-pentyl-tosylat
2.2-Dimethyl-3-methoxy-1-propyl-tosylat
2.2-Dimethyl-3-ethoxy-1-propyl-tosylat
2-Methyl-2-methoxy-1-propyl-tosylat
2.2-Dimethyl-3-fluor-1-propyl-tosylat
2.2-Dimethyl-3-Chlor-1-propyl-tosylat
2.2-Di-(Chlormethyl)-1-propyl-tosylat
2.2-Di-(Chlormethyl)-3-chlor-1-propyl-tosylat
2.2-Dimethyl-4-nitrilo-1-butyl-tosylat
3,3-Dimethyl-2-hydroxy-1-butyl-tosylat
Solketal-tosylat
2-Ethoxycarbonyl-2-methyl-1-propyl-tosylat
Hydroxypivalaldehyd-tosylat
1-Chlorpinakolin
Prenylbromid
Methallylchlorid
2-Fluormethyl-allylchlorid
Trifluorethyl-phenylsulfonat
3-Methyl-3-tosyloxymethyl-oxetan
3-Ethyl-3-mesyloxyethyl-oxetan
3-Isopropyl-3-tosyloxymethyl-oxetan
2-Methyl-2-tosyloxymethyl-pyran
Chlormethyl-trimethylsilan
3.3-Dimethyl-1-butyl-mesylat.

Die Verbindungen der allgemeinen Formel (V) sind teilweise bekannt und lassen sich nach im Prinzip bekannten Verfahren in einfacher Weise herstellen (vgl. z.B. A,. Coortin, H.R. von Tobel, G. Auerbach Helv. Chim. Acta 63, 1412 (1980)).

Als Verdünnungsmittel können bei dem erfindungsgemäßen Verfahren (A) alle inerten Solventien

eingesetzt werden. Vorzugsweise verwendbar sind Halogenkohlenwasserstoffe, wie Methylenchlorid, Chloroform, Tetrachlorkohlenstoff, Chlorbenzol und o-Dichlorbenzol und Kohlenwasserstoffe wie Toluol, Xylol und Tetralin, weiterhin cyclische Ether, wie Tetrahydrofuran und Dioxan, darüber hinaus Carbonsäureester, wie Ethylacetat und Glykolmonomethyletheracetat und auch stark polare Solventien, wie Dimethylsulfoxid, Sulfolan, N-Methylpyrrolidon, Dimethylacetamid und Dimethylformamid.

Als Säurebindemittel kommen bei der Umsetzung nach dem erfindungsgemäßen Verfahren (A) alle üblichen Säure akzeptoren in Betracht. Vorzugsweise verwendbar sind tertiäre Amine, wie Triethylamin, Pyridin und N,N-Dimethyl-anilin, ferner Erdalkalimetalloxide, wie Magnesium-und Calciumoxid, außerdem Alkali-und Erdalkali-metall-carbonate, wie Natriumcarbonat, Kaliumcarbonat und Calciumcarbonat, außerdem Alkali-und Erdalkali-metall-hydroxide, wie Lithiumhydroxid, Natriumhydroxid, Kaliumhydroxid und Calciumhydroxid, Alkali-und Erdalkalialkoholate wie Natriummethylat, Natriumethylat, Kalium-tert.-butylat und Magnesiumethylat, Alkali und Erdalkalihydride wie Natriumhydrid, Kaliumhydrid und Calciumhydrid.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -20°C und +200°C, vorzugsweise zwischen 50°C und 160°C.

Bei der Durchführung des erfindungsgemäßen Verfahrens (A) werden die Ausgangsstoffe der Formeln (II) und (III) und auch gegebenenfalls die Base im allgemeinen in angenähert äquivalenten Mengen verwendet. Es ist jedoch auch möglich die eine oder andere Reaktionskomponente in einem größeren Überschuß einzusetzen. Die Aufarbeitung erfolgt nach üblichen Methoden.

Als Verdünnungsmittel und Säurebindemittel können bei dem erfindungsgemäßen Verfahren (B) vorzugsweise diejenigen Verdünnungsmittel und Säurebindemittel verwendet werden, die auch bei dem Verfahren (A) in Betracht kommen.

Die Reaktionstemperaturen können auch bei dem erfindungsgemäßen Verfahren (B) innerhalb eines größeren Bereiches variiert werden. Im allgemeinen arbeitet man bei Temperaturen zwischen -20°C und +250°C, vorzugsweise zwischen 50°C und 180°C.

Bei der Durchführung des erfindungsgemäßen Verfahrens (B) werden die Ausgangsstoffe der allgemeinen Formeln (IV) und (V) und auch gegebenenfalls die Base im allgemeinen in angenähert äquimolaren Mengen verwendet. Es ist jedoch auch möglich die eine oder andere Reaktionskomponente in einem größeren Überschuß einzusetzen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u>Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u>Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe der allgemeinen Formel (I) mit besonders gutem Erfolg zur selektiven Bekämpfung mono-und dikotyler Unkräuter, insbesondere in monokotylen Kulturen, wie beispielsweise Weizen, im Nachauflaufverfahren einsetzen.

Bei der Anwendung im Nachauflaufverfahren können die erfindungsgemäßen Wirkstoffe allein oder in Kombination mit emulgierbaren Ölen, oberflächenaktiven Substanzen und anderen Additiven ausgebracht werden.

Darüber hinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Un krautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Als Mischpartner kommen auch in Frage:
Harnstoffe (z.B. Methabenzthiazuron, Chlortuluron, Isoproturon), Sulfonylharnstoffe (z.B. Chlorsulfuron), Triazine (z.B. Atrazin, Terbutryne, Cyanazin), Triazinone (z.B. Ethiozin, Metribuzin), Triazindione (z.B. Amethydione), Diphenylether (z.B. Bifenox), Benzonitrile (z.B. Ioxynil, Bromoxynil), Phenoxyalkancarbonsäuren (z.B. 2,4 D, 2,4 DP, MCPA, MCPP etc.), Aryloxy-oder Heteroaryloxyphenoxypropionsäuren (z.B. Illoxan, Whip, (R)-2-[4-(3,5-Dichlor-pyridyl-2-oxy)-phenoxy]-propionsäure-(trimethylsilyl)-methylester, Imidazolinone (z.B. Imazamethabenz), Bentazone und Pyridate.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie begrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Herstellungsbeispiele

Beispiel 1

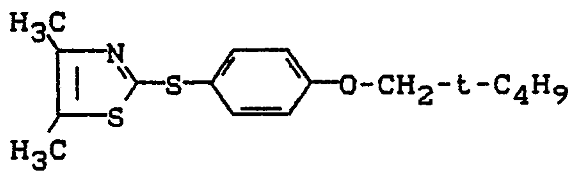

(Verfahren A)

Zu 6,12 (0,034 mol) 4-Neopentyloxythiophenol in 75 ml abs. Dimethylformamid werden bei Raumtemperatur 1,1 g (0,045 mol) 80 %iges Natriumhydrid gegeben. Nach zehnminütigem Rühren bei Raumtemperatur werden 5 g (0,034 mol) 2-Chlor-4,5-dimethylthiazol zugegeben und es wird 6 Stunden am Rückfluß erhitzt. Das Lösungsmittel wird anschließend abdestilliert und das Reaktionsgemisch in 500 ml Wasser gegeben. Nach Absaugen und Trocknen. erhält man 10 g des gewünschten Thiazols (96 % der Theorie). Fp.: 82°C.

Beispiel 2

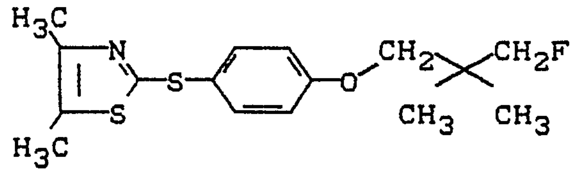

(Verfahren B)

4,74 g (20 mmol) 4-(4,5-Dimethyl-2-thiazolylthio)-phenol werden in 20 ml N-Methylpyrrolidon unter Stickstoffatmosphäre mit 2,69 g (24 mmol) Kalium-tert,-butylat versetzt und 5 Minuten verrührt. Nach der Zugabe von 4,05 g (22 mmol) 3-Fluor-neopentyl-tosylat wird 6 h auf 150°C erwärmt. Das Reaktionsgemisch wird in 150 ml 1N NaOH eingerührt, 3 x mit Toluol extrahiert, mit $MgSO_4$ getrocknet und im Wasserstrahlvakuum einrotiert. Der Rückstand wird mit Cyclohexan/ Essigester 3:1 an Kieselgel chromatographiert. Man erhält 3,68 g (56,6 %) des Thiazolylethers durch Kristallisation aus Essigester/n-Hexan vom Schmelzpunkt 85°C.

Nach den in den vorausgehenden Beispielen und in der Beschreibung angegebenen Methoden lassen sich die folgenden formelmäßig aufgeführten Verbindungen der allgemeinen Formel (I) herstellen.

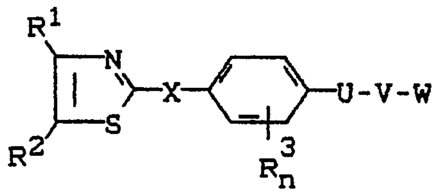

(I)

Tabelle 1

| Bsp. Nr. | $R^1$ | $R^2$ | $R^3$ | n | X | U | D | $R^4$ | $R^5$ | $R^6$ | $R^7$ | $R^8$ | Schmelzpunkt °C |
|---|---|---|---|---|---|---|---|---|---|---|---|---|---|
| 3 | $CH_3$ | $CH_3$ | H | - | O | O | C | H | H | $CH_3$ | $CH_3$ | $CH_3$ | $n_D^{20}$ :1.5321 |
| 4 | $CH_3$ | $CH_3$ | H | - | S | O | C | H | H | H | $CH_3$ | $CH_3$ | 34 |
| 5 | $CH_3$ | $CH_3$ | H | - | S | O | C | H | H | H | $CH_3$ | $C_2H_5$ | Öl |
| 6 | $CH_3$ | $CH_3$ | H | - | S | O | C | H | H | $CH_3$ | $CH_3$ | $C_2H_5$ | 31 |
| 7 | $CH_3$ | $CH_3$ | H | - | S | O | C | H | H | $CH_3$ | $CH_3$ | $C_3H_7$ | Öl |
| 8 | $CH_3$ | $CH_3$ | H | - | S | O | C | H | H | $CH_3$ | $CH_3$ | $CH_2Cl$ | 44 |
| 9 | $CH_3$ | $CH_3$ | H | - | S | O | C | H | H | $CH_3$ | $CH_3$ | $CH_2OCH_3$ | Öl |
| 10 | $CH_3$ | $CH_3$ | H | - | S | O | C | H | H | $CH_3$ | $CH_3$ | $CH_2OC_2H_5$ | 22 |
| 11 | $CH_3$ | $CH_3$ | H | - | S | O | C | H | H | $CH_3$ | $-CH_2-O-CH_2-$ | | 74 |
| 12 | $CH_3$ | $CH_3$ | H | - | S | O | C | H | H | $C_2H_5$ | $-CH_2-O-CH_2-$ | | 35 |
| 13 | $CH_3$ | $CH_3$ | H | - | S | O | C | H | H | $CH_3$ | $CH_3$ | $OCH_3$ | 30 |
| 14 | $CH_3$ | $CH_3$ | H | - | S | O | C | H | H | $CH_3$ | $-O-(CH_2)_4-$ | | 53 |
| 15 | $CH_3$ | $CH_3$ | H | - | S | O | C | H | H | F | F | F | 41 |
| 16 | $CH_3$ | $CH_3$ | H | - | S | S | C | H | H | $CH_3$ | $CH_3$ | $CH_3$ | 40 |

Beispiel 17

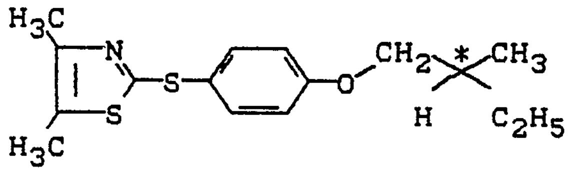

S-Enantiomer

$^{1}$H-NMR (200 MHz, $CDCl_3$) δ = 0,95 (t; 3H, $CH_2CH_3$), 1,03 (d; 3H, $\overset{*}{C}H-CH_3$), 1,29 (m; 1H, $CH_2CH_3$), 1,55 (m; 1H, $\overset{*}{C}H<$ 1,78 (m; 1H, $CH_2CH_3$), 2,21 2,27 (2s; 6H, Thiazolyl-4-$CH_3$ und 5-$CH_3$), 3,79 (ABq; 2H, O-$CH_2$- $\overset{*}{C}H$), 6,92 7,54 (AA'BB'; 4H, Ar-H).

Tabelle 1 (Fortsetzung)

| Bsp. Nr. | $R^1$ | $R^2$ | $R^3$ | n | X | U | D | $R^4$ | $R^5$ | $R^6$ | $R^7$ | $R^8$ | Schmelzpunkt (°C) |
|---|---|---|---|---|---|---|---|---|---|---|---|---|---|
| 18 | $CH_3$ | $CH_3$ | H | 0 | S | O | C | H | H | H | $CH_3$ | $C_3H_7$ | Öl |
| 19 | $CH_3$ | $CH_3$ | H | 0 | S | O | C | H | H | H | $C_2H_5$ | $C_4H_9$ | Öl |
| 20 | $CH_3$ | $CH_3$ | H | 0 | S | O | C | H | H | H | H | $C(CH_3)_3$ | Öl |
| 21 | $CH_3$ | $CH_3$ | H | 0 | S | O | C | H | H | H | H | $C(CH_3)_2OCH_3$ | Öl |
| 22 | $CH_3$ | $CH_3$ | H | 0 | S | O | C | H | H | H | $-O-(CH_2)_4-$ | | 58 |
| 23 | $CH_3$ | $CH_3$ | H | 0 | S | O | C | H | H | $CH_3$ | $-O-CH=CH-(CH_2)_2-$ | | 61 |
| 24 | $CH_3$ | $CH_3$ | H | 0 | S | O | C | H | H | $CH_3$ | $-CH_2-OC(CH_3)_2-O-CH_2-$ | | 76 |
| 25 | $CH_3$ | $CH_3$ | H | 0 | S | O | C | H | H | $CH_3$ | $-O-(CH_2)_3-O-$ | | Öl |
| 26 | $CH_3$ | $CH_3$ | H | 0 | S | O | C | H | H | $CH_3$ | $-O-CH_2-C(CH_3)_2-CH_2-O-$ | | 67 |
| 27 | $CH_3$ | $CH_3$ | H | 0 | S | O | C | H | H | $CH_3$ | $-O-CH(CH_3)-CH_2-C(CH_3)_2-O-$ | | Öl |
| 28 | $CH_3$ | $CH_3$ | H | 0 | S | O | C | H | $CH_3$ | $CH_3$ | $CH_3$ | $CH_3$ | 46 |
| 29 | $CH_3$ | $CH_3$ | 3-Cl | 1 | S | O | C | H | H | $CH_3$ | $CH_3$ | $CH_3$ | 52 |

Tabelle 2

| Bsp. Nr. | $R^1$ | $R^2$ | $R^3$ | n | X | U | $R^4$ | $R^5$ | $R^9$ | $R^{10}$ | $R^{11}$ | Schmelzpunkt (°C) |
|---|---|---|---|---|---|---|---|---|---|---|---|---|
| 30 | $CH_3$ | $CH_3$ | H | O | S | O | H | H | H | H | $CH_3$ | 31 |
| 31 | $CH_3$ | $CH_3$ | H | O | S | O | H | H | H | H | $CH_2F$ | Öl |

Tabelle 3

| Bsp. Nr. | $R^1$ | $R^2$ | $R^3$ | n | X | U | $R^4$ | $R^5$ | E | $R^{12}$ | Schmelzpunkt (°C) |
|---|---|---|---|---|---|---|---|---|---|---|---|
| 32 | $CH_3$ | $CH_3$ | H | O | S | O | H | H | O | $CH_3$ | 47 |
| 33 | $CH_3$ | $CH_3$ | H | O | S | O | H | H | O | $-C(CH_3)_3$ | 109 |

| Bsp. Nr. | Verbindungen | Schmelzpunkt °C |
|---|---|---|
| 34 | 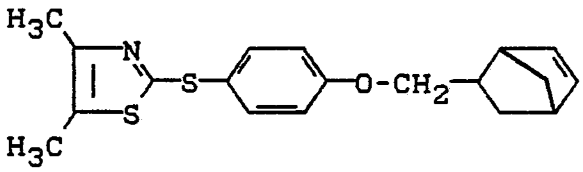 | 41 |
| 35 | 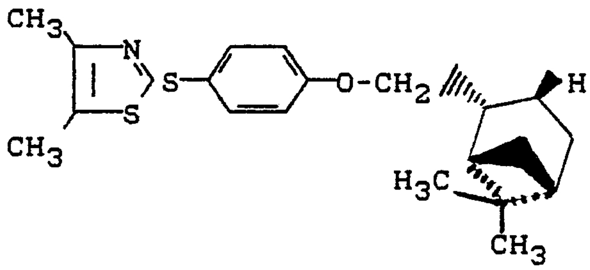 | 76 |
| 36 | 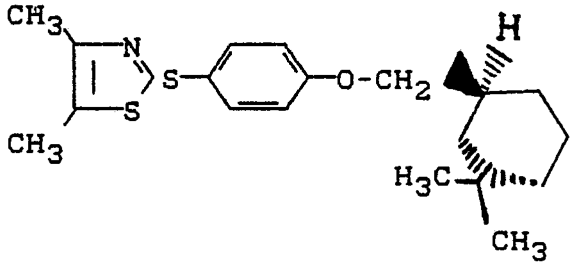 | 69 |

Herstellung der Ausgangsprodukte:

Beispiel a)

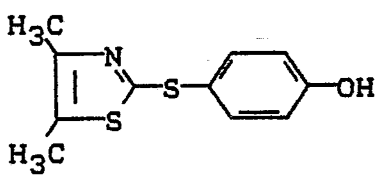

(Verfahren C)

40 g (0,61 mol) gep. KOH ca. 85 %ig wird in 366 ml N-Methylpyrrolidon unter Stickstoffatmosphäre vorgelegt. Dazu tropft man bei 60°C 71 g (0,61 mol) 4-Mercaptophenol. Bei 100°C wurden anschließend 90 g (0,61 mol) 4,5-Dimethyl-2-chlorthiazol zugetropft und 2 h bei 100°C nachgerührt. Im Hochvakuum werden ca. 2/3 des N-Methylpyrrolidons abdestilliert, der Rückstand auf Wasser gegossen und die Lösung auf pH 7 eingestellt. Man extrahiert mit Chloroform, trocknet und rotiert im WSV ein. Nach dem Umkristallisieren aus Methanol erhält man 103 g (75 % der Theorie) 4,5-Dimethylthiazolylthio)-phenol vom Schmp. 145°C.

Verwendungsbeispiel

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen beispielsweise die erfindungsgemäßen Verbindungen der Herstellungsbeispiele 1, 8,9 und 13 eine sehr gute selektive herbizide Wirkung gegen Unkräuter wie beispielsweise Abutilon, Datura oder Stellaria.

**Ansprüche**

1. Thiazolylderivate der allgemeinen Formel (I),

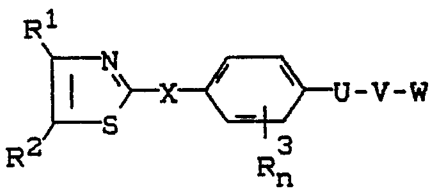

(I)

in welcher
$R^1$ für Wasserstoff oder Alkyl steht,
$R^2$ für Wasserstoff oder Alkyl steht,
mit der Maßgabe, daß $R^1$ und $R^2$ nicht gleichzeitig für Wasserstoff stehen,
X für Sauerstoff oder Schwefel steht,
$R^3$ für Wasserstoff, Halogen, Nitro, Amino, Cyano, Alkyl, Halogenalkyl, Alkoxy oder Halogenalkoxy steht, wobei die Reste $R^3$ gleich oder verschieden sein können,
n für eine Zahl von 1 bis 4 steht,
U für Sauerstoff oder Schwefel steht,
V für

eine 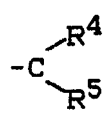 -Gruppe

steht, wobei
$R^4$ und $R^5$ unabhängig voneinander für Wasserstoff, Alkyl, Alkoxyalkyl oder Halogenalkyl steht,
W für einen Rest

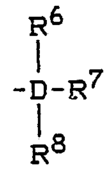 , 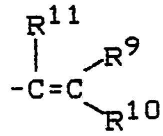 oder 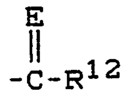

steht, wobei

D für Kohlenstoff oder Silicium steht,

E für Sauerstoff oder Schwefel steht,

$R^6$, $R^{11}$ und $R^{12}$ unabhängig voneinander für Wasserstoff, Halogen, Alkyl oder Halogenalkyl stehen,

$R^7$ für Wasserstoff, Halogen, gegebenenfalls durch Halogen substituiertes Alkyl, Alkoxy, Alkoxyalkyl oder Alkylthio steht,

$R^8$ für Halogen, einen gegebenenfalls durch Halogen substituierten gesättigten oder ungesättigten aliphatischen Rest mit 1 bis 6 Kohlenstoffatomen, Alkoxy, Alkoxyalkyl, Alkylthio, Formyl, Alkylcarbonyl, Alkoxycarbonyl, offenkettiges oder cyclisches Acetal, offenkettiges oder cyclisches Thioacetal steht, oder

$R^7$ und $R^8$ gemeinsam mit dem Kohlenstoff, an das sie gebunden sind, einen 3 bis 8 gliedrigen Ring bilden, der außer Kohlenstoff auch Sauerstoff und/oder Schwefel und/oder Stickstoff als Ringglieder enthalten kann und der gegebenenfalls durch Halogen, Alkyl, Alkoxy oder Alkoxyalkyl substituiert sein kann und welcher eine oder mehrere Doppelbindungen enthalten kann, oder

$R^6$, $R^7$ und $R^8$ gemeinsam mit dem Kohlenstoffatom, an das sie gebunden sind, für einen Rest der Struktur

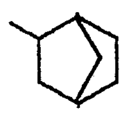 oder 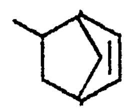

stehen, und

$R^9$ und $R^{10}$ unabhängig voneinander für Wasserstoff, Halogen, Alkyl oder Halogenalkyl stehen,

sowie deren optisch aktive Verbindungen.

2. Verbindungen der allgemeinen Formel (I) gemäß Anspruch 1, worin

$R^1$ für Wasserstoff oder $C_1$-$C_4$-Alkyl steht,

$R^2$ für Wasserstoff oder $C_1$-$C_4$-Alkyl steht,

mit der Maßgabe, daß $R^1$ und $R^2$ nicht gleichzeitig für Wasserstoff stehen,

X für Sauerstoff oder Schwefel steht,

$R^3$ für Wasserstoff, Halogen, $C_1$-$C_4$-Alkyl, $C_1$-$C_4$-Halogenalkyl, $C_1$-$C_4$-Alkoxy oder Halogen-$C_1$-$C_4$-alkoxy steht, wobei die Reste $R^3$ gleich oder verschieden sein können,

n für eine Zahl von 1 bis 4 steht,

U für Sauerstoff oder Schwefel steht,

V für

eine 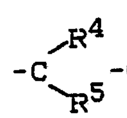 -Gruppe

steht, wobei

$R^4$ und $R^5$ unabhängig voneinander jeweils für Wasserstoff, $C_1$-$C_4$-Alkyl, $C_1$-$C_4$-Alkoxy-$C_1$-$C_4$-alkyl oder Halogen-$C_1$-$C_4$-alkyl stehen,

W für einen Rest

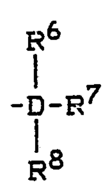 , 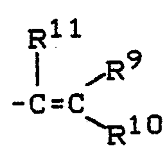 oder 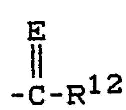

steht, wobei

D für Kohlenstoff oder Silicium steht,

E für Sauerstoff oder Schwefel steht,

$R^6$, $R^{11}$ und $R^{12}$ unabhängig voneinander für Wasserstoff, Halogen, $C_1$-$C_6$-Alkyl oder Halogen-$C_1$-$C_6$-alkyl stehen,

$R^7$ für Wasserstoff, Halogen, $C_1$-$C_6$-Alkyl, $C_1$-$C_6$-Alkoxy, $C_1$-$C_6$-Alkoxy-$C_1$-$C_6$-alkyl, $C_1$-$C_6$-Alkylthio oder Halogen-$C_1$-$C_6$-alkyl steht,

$R^8$ für Halogen, $C_1$-$C_6$-Alkyl, $C_1$-$C_6$-Alkoxy, $C_1$-$C_6$-Alkoxy-$C_1$-$C_6$-alkyl, $C_1$-$C_6$-Alkylthio, Formyl, $C_1$-$C_6$-Alkylcarbonyl, $C_1$-$C_6$-Alkoxycarbonyl, $C_2$-$C_6$-Alkenyl, $C_2$-$C_6$-Alkinyl, Halogen-$C_1$-$C_6$-alkyl, Halogen-$C_2$-$C_6$-alkenyl, offenkettiges Acetal, cyclisches Acetal mit 5 oder 6 Ringgliedern, offenkettiges Thioacetal oder cyclisches Thioacetal mit 5 oder 6 Ringgliedern steht, oder

$R^7$ und $R^8$ gemeinsam mit dem Kohlenstoffatom, an das sie gebunden sind, einen 5-oder 6-gliedrigen Ring bilden, der außer Kohlenstoff auch 1 oder 2 Sauerstoffatome und/oder Schwefelatome und/oder Stickstoffatome als Ringglieder enthalten kann und der durch Fluor, Chlor, Brom, Jod, $C_1$-$C_4$-Alkyl, $C_1$-$C_4$-Alkoxy und/oder $C_1$-$C_4$-Alkoxy-$C_1$-$C_4$-alkyl substituiert sein kann und welcher eine oder zwei Doppelbindungen enthalten kann oder

$R^6$, $R^7$ und $R^8$ gemeinsam mit dem Kohlenstoffatom, an das sie gebunden sind, für einen Rest der Struktur

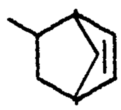 oder 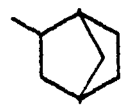

stehen, und

$R^9$ und $R^{10}$ unabhängig voneinander für Wasserstoff, Halogen, $C_1$-$C_6$-Alkyl oder Halogen-$C_1$-$C_6$-alkyl stehen, sowie deren optisch aktive Verbindungen.

3. Verbindungen der allgemeinen Formel (I) gemäß Anspruch 1, worin

$R^1$ für Wasserstoff, Methyl oder Ethyl steht,

$R^2$ für Wasserstoff, Methyl oder Ethyl steht,

mit der Maßgabe, daß $R^1$ und $R^2$ nicth gleichzeitig für Wasserstoff stehen,

X für Sauerstoff oder Schwefel steht,

$R^3$ für Wasserstoff, Halogen, Methyl, Ethyl, Methoxy, Ethoxy, Halogen-$C_1$-$C_2$-alkyl oder Halogen-$C_1$-$C_2$-alkoxy steht,

wobei die Reste $R^3$ gleich oder verschieden sein können,

n für eine Zahl von 1 bis 4 steht,

U für Sauerstoff oder Schwefel steht,

V für

eine 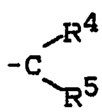 -Gruppe

steht, wobei

$R^4$ und $R^5$ unabhängig voneinander jeweils für Wasserstoff, Methyl, Ethyl, Methoxymethyl, Methoxyethyl, Ethoxymethyl, Ethoxyethyl oder Halogen-$C_1$-$C_2$-alkyl stehen,

W für einen Rest

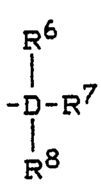 , 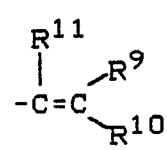 oder 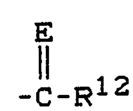

steht, wobei

D für Kohlenstoff oder Silicium steht,

E für Sauerstoff oder Schwefel steht,
$R^6$, $R^{11}$ und $R^{12}$ unabhängig voneinander für Wasserstoff, Fluor, Chlor, Brom, Jod, $C_1$-$C_4$-Alkyl oder Halogen-$C_1$-$C_4$-alkyl steht,
$R^7$ für Wasserstoff, Fluor, Chlor, Brom, Jod, $C_1$-$C_4$-Alkyl, $C_1$-$C_4$-Alkoxy, $C_1$-$C_4$-Alkoxy-$C_1$-$C_4$-alkyl, $C_1$-$C_4$-Alkylthio oder Halogen-$C_1$-$C_4$-alkyl steht,
$R^8$ für Fluor, Chlor, Brom, Jod, $C_1$-$C_4$-Alkyl, $C_1$-$C_4$-Alkoxy, $C_1$-$C_4$-Alkoxy-$C_1$-$C_4$-alkyl, $C_1$-$C_4$-Alkylthio, Formyl, $C_1$-$C_4$-Alkylcarbonyl, $C_1$-$C_4$-Alkoxycarbonyl, $C_2$-$C_4$-Alkenyl, $C_2$-$C_4$-Alkinyl, Halogen-$C_1$-$C_4$-alkyl, Halogen-$C_2$-$C_4$-alkenyl, offenkettiges Acetal, cyclisches Acetal mit 5 oder 6 Ringgliedern, offenkettiges Thioacetal oder cyclisches Thioacetal mit 5 oder 6 Ringgliedern steht oder
$R^7$ und $R^8$ gemeinsam mit dem Kohlenstoffatom, an das sie gebunden sind, einen 5-oder 6-gliedrigen Ring bilden, der außer Kohlenstoff auch 1 oder 2 Sauerstoffatome und/oder Schwefelatome und/oder Stickstoffatome als Ringglieder enthalten kann und der durch Fluor, Chlor, Brom und Jod, Methyl, Ethyl, Methoxy, Ethoxy, Methoxymethyl, Methoxyethyl, Ethoxymethyl oder Ethoxyethyl substituiert sein kann und welcher eine oder zwei Doppelbindungen enthalten kann, oder
$R^6$, $R^7$ und $R^8$ gemeinsam mit dem Kohlenstoffatom, an das sie gebunden sind, für einen Rest der Struktur

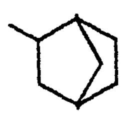 oder 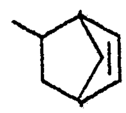

stehen, und
$R^9$ und $R^{10}$ unabhängig voneinander für Wasserstoff, Halogen, $C_1$-$C_2$-Alkyl oder Halogen-$C_1$-$C_2$-alkyl stehen, sowie deren optisch aktive Verbindungen.

4. Verfahren zur Herstellung von Thiazolylderivaten der allgemeinen Formel (I),

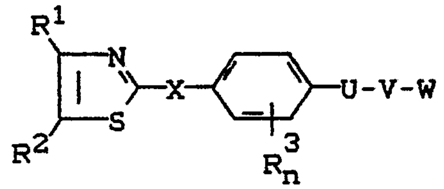 (I)

in welcher
$R^1$ für Wasserstoff oder Alkyl steht,
$R^2$ für Wasserstoff oder Alkyl steht,
mit der Maßgabe, daß $R^1$ und $R^2$ nicht gleichzeitig für Wasserstoff stehen,
X für Sauerstoff oder Schwefel steht,
$R^3$ für Wasserstoff, Halogen, Nitro, Amino, Cyano, Alkyl, Halogenalkyl, Alkoxy oder Halogenalkoxy steht, wobei die Reste $R^3$ gleich oder verschieden sein können,
n für eine Zahl von 1 bis 4 steht
U für Sauerstoff oder Schwefel steht,
V für

eine 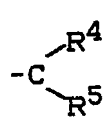 -Gruppe

steht, wobei
$R^4$ und $R^5$ unabhängig voneinander für Wasserstoff, Alkyl, Alkoxyalkyl oder Halogenalkyl steht,
W für einen Rest

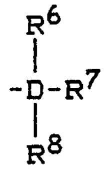 , 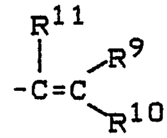 oder 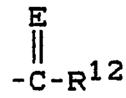

steht, wobei
D für Kohlenstoff oder Silicium steht,
E für Sauerstoff oder Schwefel steht,
$R^6$, $R^{11}$ und $R^{12}$ unabhängig voneinander für Wasserstoff, Halogen, Alkyl oder Halogenalkyl stehen,
$R^7$ für Wasserstoff, Halogen, gegebenenfalls durch Halogen substituiertes Alkyl, Alkoxy, Alkoxyalkyl oder Alkylthio steht,
$R^8$ für Halogen, einen gegebenenfalls durch Halogen substituierten gesättigten oder ungesättigten aliphatischen Rest mit 1 bis 6 Kohlenstoffatomen, Alkoxy, Alkoxyalkyl, Formyl, Alkylcarbonyl, Alkoxycarbonyl, Alkylthio, offenkettiges oder cyclisches Acetal, offenkettiges oder cyclisches Thioacetal steht, oder
$R^7$ und $R^8$ gemeinsam mit dem Kohlenstoff, an das sie gebunden sind, einen 3 bis 8 gliedrigen Ring bilden, der außer Kohlenstoff auch 1 oder 2 Sauerstoffatome und/oder Schwefelatome und/oder Stickstoffatome als Ringglieder enthalten kann und der gegebenenfalls durch Halogen, Alkyl, Alkoxy oder Alkoxyalkyl substituiert sein kann und welcher eine oder mehrere Doppelbindungen enthalten kann, oder
$R^6$, $R^7$ und $R^8$ gemeinsam mit dem Kohlenstoffatom, an das sie gebunden sind, für einen Rest der Struktur

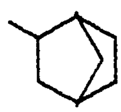 oder 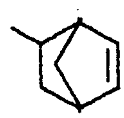

stehen, und
$R^9$ und $R^{10}$ unabhängig voneinander für Wasserstoff, Halogen, Alkyl oder Halogenalkyl stehen,
sowie deren optisch aktive Verbindungen,
dadurch gekennzeichnet, daß man
A) Verbindungen der allgemeinen Formel (II),

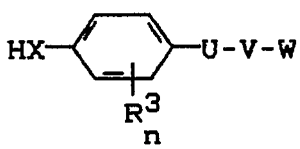 (II)

in welcher
X, $R^3$, U, V, W und n die oben angegebene Bedeutung haben,
mit Verbindungen der allgemeinen Formel (III),

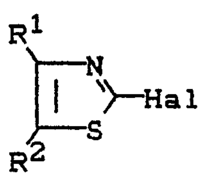 (III)

in welcher
$R^1$ und $R^2$ die obengenannte Bedeutung haben und
Hal für Halogen steht,
in Gegenwart eines Verdünnungsmittels und gegebenenfalls in Gegenwart eines Säurebindemittels umsetzt, oder daß man
B) Verbindungen der allgemeinen Formel (IV),

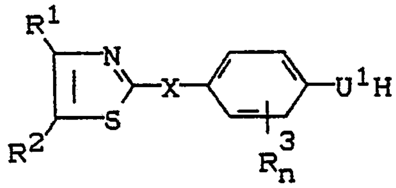

(IV)

in welcher
$R^1$, $R^2$, X, $R^3$ und n die oben angegebene Bedeutung haben und
$U^1$ Sauerstoff bedeutet,
mit Verbindungen der allgemeinen Formel (V),

L - V - W (V)

in welcher
V und W die oben angegebene Bedeutung haben und
L für Halogen, Alkylsulfonyloxy mit jeweils 1 bis 4 Kohlenstoffatomen, Halogenalkylsulfonyloxy mit jeweils 1 bis 4 Kohlenstoffatomen oder Phenyl-oder substituiertes Phenylsulfonyloxy steht,
in Gegenwart eines Verdünnungsmittels und gegebenenfalls in Gegenwart eines Säurebindemittels umsetzt.

5. Verbindungen der allgemeinen Formel (IV ),

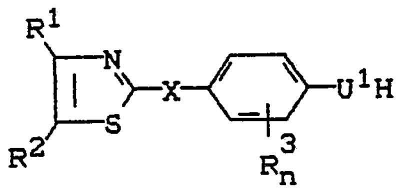

(IV )

in welcher
$R^1$ für Wasserstoff oder Alkyl steht,
$R^2$ für Wasserstoff oder Alkyl steht,
mit der Maßgabe, daß $R^1$ und $R^2$ nicht gleichzeitig für Wasserstoff stehen,
X für Sauerstoff oder Schwefel steht,
$R^3$ für Wasserstoff, Halogen, Nitro, Amino, Cyano, Alkyl, Halogenalkyl, Alkoxy oder Halogenalkoxy steht, wobei die Reste $R^3$ gleich oder verschieden sein können,
n für eine Zahl von 1 bis 4 steht, und
$U^1$ für Sauerstoff steht.

6. Verfahren zur Herstellung von Verbindungen der allgemeinen Formel (IV ),

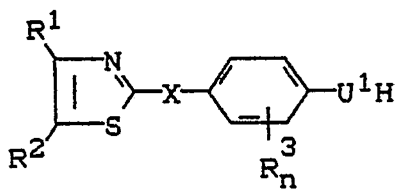

(IV )

in welcher
$R^1$ für Wasserstoff oder Alkyl steht,
$R^2$ für Wasserstoff oder Alkyl steht,
mit der Maßgabe, daß $R^1$ und $R^2$ nicht gleichzeitig für Wasserstoff stehen,
X für Sauerstoff oder Schwefel steht,
$R^3$ für Wasserstoff, Halogen, Nitro, Amino, Cyano, Alkyl, Halogenalkyl, Alkoxy oder Halogenalkoxy steht, wobei die Reste $R^3$ gleich oder verschieden sein können,
n für eine Zahl von 1-4 steht, und
$U^1$ für Sauerstoff steht,
dadurch gekennzeichnet, daß man 2-Halogen-thiazol-Derivate der allgemeinen Formel (III),

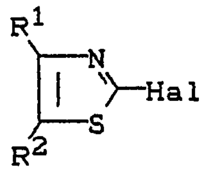

(III)

in welcher
$R^1$ und $R^2$ die obengenannte Bedeutung haben und
Hal für Halogen, insbesondere Fluor, Chlor und Brom steht,
mit Verbindungen der allgemeinen Formel (XIV),

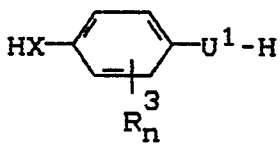

(XIV)

in welcher
$R^3$, $U^1$, X und n die oben angegebene Bedeutung haben,
in Gegenwart eines Lösungsmittels und in Gegenwart eines Säurebindemittels umsetzt.

7. Herbizide Mittel, gekennzeichnet durch einen Gehalt an mindestens einem Thiazolylderivat der allgemeinen Formel (I) gemäß den Ansprüchen 1 bis 4.

8. Verfahren zur Bekämpfung von Unkräutern, dadurch gekennzeichnet, daß man Thiazolylderivate der allgemeinen Formel (I) gemäß den Ansprüchen 1 bis 4 auf die Unkräuter und/oder ihren Lebensraum einwirken läßt.

9. Verwendung von Thiazolylderivaten der allgemeinen Formel (I) gemäß den Ansprüchen 1 bis 4 zur Bekämpfung von Unkräutern.

10. Verfahren zur Herstellung von herbiziden Mitteln, dadurch gekennzeichnet, daß man Thiazolylderivate der allgemeinen Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 88 10 3118

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CH-A- 639 963 (SHIONOGI)<br>* Ansprüche; Seite 7 *<br>--- | 1-3 | C 07 D 277/34<br>C 07 D 277/36<br>A 01 N 43/78 |
| A | GB-A-1 316 574 (COALITE AND CHEMICAL PRODUCTS LTD)<br>--- | 1-3 | |
| D,A | CHEMICAL ABSTRACTS, Band 70, Nr. 9, 3. März 1969, Seite 349, Zusammenfassung Nr. 37750r, Columbus, Ohio, US; T. JOJIMA et al.: "Syntheses of pyridazine derivatives as herbicides. III. Preparation of 3- and 4-phenoxypyridazines", & AGR. BIOL. CHEM. (TOKYO) 1968, 32(11), 1376-81<br>* Zusammenfassung *<br>----- | 1,7-9 | |

| RECHERCHIERTE SACHGEBIETE (Int. Cl.4) |
|---|
| C 07 D 277/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 15-06-1988 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)